# EUROPÄISCHE PATENTSCHRIFT

(11) **EP 2 288 591 B1**
(45) Veröffentlichungstag und Bekanntmachung des Hinweises auf die Patenterteilung: **20.08.2014**
(21) Anmeldenummer: 09765702.7
(22) Anmeldetag: 20.05.2009
(51) Int. Cl.: C07C 209/78, C07C 209/84, C07C 211/50

(54) **VERFAHREN ZUR HERSTELLUNG VON DIPHENYLMETHANDIAMIN**
METHOD FOR PRODUCING DIPHENYLMETHANE DIAMINE
PROCEDE DE PREPARATION DE DIAMINE DE DIPHENYLMETHANE

(30) Priorität: 26.05.2008 EP 08156927
(43) Veröffentlichungstag der Anmeldung: 02.03.2011
(73) Patentinhaber: BASF SE, 67056 Ludwigshafen (DE)
(72) Erfinder: JACOBS, Johannes, 4641 PE Ossendrecht (NL)
(86) Internationale Anmeldenummer: PCT/EP2009/056118
(87) Internationale Veröffentlichungsnummer: WO 2009/153122

(56) Entgegenhaltungen:
- WO-A-99/40059
- ANONYMOUSLY: "Using toluene in an agitated column to continuously extract impurities contained in aqueous effluent from a diamino-diphenyl methane (DADPM) manufacturing process" RESEARCH DISCLOSURE, Nr. 487083, 1. November 2004 (2004-11-01), XP002553389
- DATABASE CA [Online] CHEMICAL ABSTRACTS SERVICE, COLUMBUS, OHIO, US; GACA, JERZY ET AL: "Treatment of wastewater from the manufacture of 4,4'-diaminodiphenylmethane" XP002553443 gefunden im STN Database accession no. 133:197806 & PRZEMYSL CHEMICZNY , 79(5), 165-167 CODEN: PRCHAB; ISSN: 0033-2496, 2000,

## Beschreibung

Die Herstellung von Diphenylmethandiamin (MDA) durch Umsetzung von Anilin mit Formaldehyd in Anwesenheit einer Säure ist bekannt und vielfach beschrieben. In der Praxis fällt das so hergestellte Diphenylmethandiamin stets im Gemisch mit höherkondensierten Polyphenylenpolymethylenpolyaminen an. Im folgenden wird unter "MDA" das Gemisch aus zweikernigem Diphenylmethandiamin und höherkondensierten Polyphenylenpolymethylenpolyaminen verstanden werden.

Das MDA wird in der Technik zumeist durch Umsetzung mit Phosgen zu Dipenylmethandiisocyanat MDI umgesetzt.

In der Technik erfolgt die Herstellung des MDA, wie beschrieben, durch Umsetzung von Anilin mit Formaldehyd in Anwesenheit einer Säure. Als Säure wird üblicherweise Salzsäure eingesetzt. Derartige Verfahren sind allgemein bekannt und beispielsweise im Kunststoffhandbuch, Band 7, Polyurethane, Carl Hanser Verlag München Wien, 3. Auflage, 1993, Seiten 76 bis 86, sowie in einer großen Zahl von Patentanmeldungen, beispielsweise WO 99/40059, beschrieben.

Durch die Variation des Verhältnisses von Säure zu Anilin und von Formaldehyd zu Anilin kann der Anteil des 2-Kern-Produkts im MDA je nach Wunsch eingestellt werden.

Nach der Kondensation erfolgt die Neutralisation des sauren Katalysators, zumeist durch Zusatz von Natronlauge. Dabei kommt es zu Trennung der organischen Phase, die überwiegend MDA enthält, von der wässrigen Phase, die eine wässrige Lösung des bei der Neutralisation anfallenden Salzes ist.

Die organische Phase wird üblicherweise aufgearbeitet und mit Phosgen zum entsprechenden Isocyanat umgesetzt. Die Aufarbeitung erfolgt üblicherweise durch Entfernung von Verunreinigungen, beispielsweise dem bei der Neutralisation entstehenden Salz, beispielsweise durch Waschen mit Wasser, und nachfolgende Destillation zur Entfernung des Wassers.

Die wässrige Phase enthält zumeist noch organische Bestandteile. Ein Maß für die Angabe der organischen Bestandteile ist der Gehalt an organisch gebundenem Kohlenstoff, in der Technik zumeist als TOC bezeichnet.

Diese organischen Bestandteile können bei der Verwendung der Salzlösung, beispiels-weise bei der Abtrennung der Salze oder der Gewinnung von Chlor aus der Salzlösung, Probleme bereiten.

In der Praxis erfolgt die Abtrennung der organischen Bestandteile beispielsweise durch Waschen oder durch Extraktion. Die Anteile an organischen Verbindungen sind jedoch meist noch zu hoch.

So beschreibt RD 487083 die Extraktion der Salzlösung mit Toluol, um die dort enthaltnen Wertprodukte Anilin und MDA zurückzugewinnen. Besondere Anforderungen an die Salzlösung werden nicht beschrieben.

In Chemical Abstracts, Service, Columbus, Ohio, Gaca, Jerzy et al.: "Treatment of wastewater from the manufacture of 4,4'-diaminodiphenylmethane, XP002553443, wird ein Verfahren zur Aufarbeitung der Salzlösung beschrieben. Dort wird aus der Salzlösung das Anilin und MDA entfernt und in das Verfahren zurückgeführt. Dazu wird die Salzlösung einer Extraktion und danach einer Adsorption an Aktivkohle unterworfen.

Die dabei erhaltene Salzlösung enthält nach der Behandlung noch eine große Menge an organischen Anteilen. Eine Salzlösung mit einer so großen Menge an organischen Anteilen ist für eine Elektrolyse nicht geeignet.

Es war die Aufgabe der vorliegenden Erfindung, ein Verfahren zur Herstellung von MDA zu entwickeln, bei dem eine wässrige Phase entsteht, die einen sehr geringen Gehalt an organischen Verbindungen aufweist. Insbesondere sollte der Gehalt an organisch gebundenem Kohlenstoff (TOC) kleiner 25 ppm betragen.

Die Aufgabe konnte durch eine mehrstufige Aufarbeitung gelöst werden.

Gegenstand der Erfindung ist demzufolge ein Verfahren zur Herstellung von Mischungen von Diphenylmethandiamin und Polyphenylenpolymethylenpolyaminen, umfassend die Schritte,
a) Umsetzung von Anilin mit Formaldehyd in Anwesenheit von Salzsäure,
b) Neutralisation der in Schritt a) entstehenden Reaktionsmischung.
c) Trennung der organischen Phase von der wässrigen Phase
d) Aufarbeitung der organischen Phase
e) Aufarbeitung der wässrigen Phase,
dadurch gekennzeichnet, dass der Schritt e) mindestens die Schritte
e1) Extraktion der wässrigen Phase mit einem organischen Lösungsmittel,
e2) Strippen der in Schritt e1) erhaltenen wässrigen Phase
e3) Absorption der aus Schritt e2) erhaltenen Lösung.

Vorzugsweise wird als Säure eine Mineralsäure, insbesondere Salzsäure, eingesetzt.

Die Herstellung des MDA in Schritt a) erfolgt, wie oben beschrieben, durch Umsetzung von Anilin mit Formaldehyd in Gegenwart von Säuren als Katalysatoren. Derartige Verfahren sind allgemein bekannt und beispielsweise im Kunststoffhandbuch, Band 7, Polyurethane, Carl Hanser Verlag München Wien, 3. Auflage, 1993, Seiten 76 bis 86, sowie in einer großen Zahl von Patentanmeldungen, beispielsweise WO 99/40059, beschrieben.

An Stelle von oder im Gemisch mit Formaldehyd kann auch mindestens eine Formaldehyd abspaltende Verbindung eingesetzt werden. Insbesondere wird der Formaldehyd als wässrige Formalinlösung, alkoholische Formalinlösung, Halbacetal, Methylenimin eines primären Amins oder N,N'-Methylendiamin eines primären oder sekundären Amins sowie Paraformaldehyd eingesetzt.

Das erfindungsgemäße Verfahren kann kontinuierlich, halbkontinuierlich oder batchweise, vorzugsweise kontinuierlich oder halbkontinuierlich, durchgeführt werden.

Bei der kontinuierlichen Fahrweise werden die Reaktanden in dem gewünschten Verhältnis zueinander in einen Reaktor eindosiert und diesem Reaktor eine dem Zustrom gleiche Menge an Reaktionsprodukt entnommen. Als Reaktoren kommen beispielsweise Rohrreaktoren zum Einsatz. Bei der batchweisen oder halbkontinuierlichen Fahrweise werden die Reaktanden in einen vorzugsweise mit einem Rührer und/oder einem Umpumpkreis versehenen Batchreaktor dosiert, aus dem das ausreagierte Reaktionsprodukt entnommen und der Aufarbeitung zugeführt wird.

Das erfindungsgemäße Verfahren wird vorzugsweise bei einem molaren Verhältnis von Anilin zu Formaldehyd größer 2 durchgeführt. Das molare Verhältnis von Säure zu Anilin ist vorzugsweise größer 0,05. Bei diesen Verhältnissen kommt es zu einer verstärkten Bildung der jeweiligen Zweikernprodukte in der Reaktionsmischung.

Die Reaktion wird vorzugsweise bei einer Temperatur im Bereich zwischen 0 und 200 °C, vorzugsweise zwischen 20 und 150 °C und insbesondere zwischen 40 und 120 °C durchgeführt. Es hat sich gezeigt, dass mit der Erhöhung der Temperatur der Anteil der 2,2'- und 2,4'-Isomeren im Reaktionsprodukt ansteigt.

Der Druck bei der Umsetzung beträgt 0,1 - 50, bevorzugt 1 - 10 bar absolut.

Bei der batchweisen und halbkontinuierlichen Durchführung der Reaktion kann nach der vollständigen Dosierung der Einsatzstoffe das Reaktionsgemisch einer sogenannten Alterung unterzogen werden. Dazu wird das Reaktionsgemisch im Reaktor belassen oder in einen anderen, vorzugsweise gerührten Reaktor überführt. Dabei liegt die Temperatur des Reaktionsgemisches vorzugsweise über 75 °C, insbesondere in einem Bereich zwischen 110 und 150 °C.

An die Herstellung in Schritt a) schließt sich die Neutralisation b) des Reaktionsgemischs an. Dazu wird der Reaktionsmischung eine Lauge, vorzugsweise Natronlauge zugesetzt. Die Zusammenführung der Lauge mit der Reaktionsmischung erfolgt üblicherweise in einem geeigneten Mischapparat wie einem Rührkessel, einem Rohr, das gegebenenfalls mit statischen Mischelementen versehen ist, oder anderen Apparaten. Die Zugabe der Lauge bewirkt eine Neutralisation der Reaktionsmischung und dadurch die Bildung zweier nichtmischbarer Phasen, der wässrigen und der organischen Phase. Die Neutralisation erfolgt bei einer mittleren Temperatur von 40 bis 120 °C und einem Druck von 1 bis 10 bar absolut.

Das Gemisch aus Schritt b) liegt, wie beschrieben, in einer organischen und einer wässrigen Phase vor. Diese Phasen werden in Schritt c) getrennt. Die Phasen können beispielsweise durch Dekantieren voneinander getrennt werden. Danach werden die jeweiligen Phasen separat aufgearbeitet.

Die in Schritt c) abgetrennte organische Phase, die überwiegend aus MDA mit Resten von Wasser, Ammoniak und den Einsatzprodukten für die Herstellung des MDA besteht, wird in Schritt d) aufgearbeitet. Dies erfolgt beispielsweise durch ein- oder mehrmaliges Waschen mit Wasser oder bevorzugt durch mehrfache Destillation zur Abtrennung von beispielsweise Anilin und Wasser.

Das nach dem erfindungsgemäßen Verfahren hergestellte MDA wird üblicherweise mit Phosgen zu MDI umgesetzt. Derartige Verfahren sind allgemein bekannt und vielfach beschrieben, beispielsweise im Kunststoffhandbuch, Band 7, Polyurethane, Carl Hanser Verlag München Wien, 3. Auflage, 1993, Seiten 76 bis 86, sowie in einer großen Zahl von Patentanmeldungen, beispielsweise WO 99/40059 oder WO 99/54289.

Die wässrige Phase, die im wesentlichen aus Wasser, dem darin gelösten Salz der als Katalysator eingesetzten Säure, sowie Spuren der Einsatzstoffe Anilin und Formaldehyd und auch des Endprodukts MDA und weiteren organischen Verbindungen besteht, wird in Schritt d) weiter aufgearbeitet.

Dazu wird diese zunächst einer Extraktion mit einem organischen Lösungsmittel e1) unterworfen. Bei den organischen Lösungsmitteln handelt es sich zumeist um solche, die mit den Bestandteilen der wässrigen Phase nicht reaktiv sind. Vorzugsweise werden aromatische Lösungsmittel, beispielsweise Toluol eingesetzt.

Dabei wird zumeist noch in der wässrigen Phase enthaltendes Anilin und MDA abgetrennt. Die Extraktion erfolgt vorzugsweise in einer Extraktionskolonne. Die wässrige Phase wird der weiteren Bearbeitung zugeführt, die organische Phase wird getrennt, wobei das Toluol in die Extraktion zurückgeführt und das abgetrennte Anilin und TDA in den Prozess zurückgeführt wird.

Die Extraktion erfolgt vorzugsweise bei einer Temperatur von 50 - 100 °C. Die Extraktion kann bei Normaldruck oder bei Über- oder Unterdruck, vorzugsweise bei Normaldruck durchgeführt werden.

Die wässrige Phase aus Schritt e1) wird danach einem Strippen e2) unterworfen. Das Strippen kann mit Wasserdampf oder einem Inertgas, beispielsweise Stickstoff, erfolgen. Vorzugsweise wird Wasserdampf eingesetzt.

Das Strippen erfolgt vorzugsweise bei einer Temperatur im Bereich von 150-50 °C, vorzugsweise 110 - 70°C. Vorzugsweise erfolgt das Strippen bei Normaldruck oder leichtem Über- oder Unterdruck, insbesondere bei Normaldruck.

An das Strippen schließt sich eine Adsorption an. Als Adsorptionsmittel können Zeolithe, organische Adsorberharze oder Aktivkohle verwendet werden. Vorzugsweise wird Aktivkohle eingesetzt. Vorzugsweise ist das Adsorptionsmittel als Festbett ausgestaltet. Dabei ist es bevorzugt, mehrere dieser Festbettadsorber parallel zu schalten.

Die Regenerierung des Adsorptionsmittels kann beispielsweise durch thermische Behandlung erfolgen.

Nach der Adsorption hat die wässrige Phase zumeist einen Gehalt an organischen Verbindungen von höchstens 25 ppm.

In einer bevorzugten Ausführungsform der Erfindung kann sich an die Adsorption noch eine oxidative Behandlung der wässrigen Phase anschließen. Diese kann beispielsweise durch Behandlung mit Ozon, mit Peroxiden, insbesondere Wasserstoffperoxid, oder mit Hypochloriden erfolgen. Dadurch kann der Gehalt an organischen Bestandteilen weiter reduziert werden.

In einer weiteren Ausführungsform der Erfindung kann nach Schritt e3) eine Kristallisation des gelösten Salzes erfolgen. Die Kristallisation kann einen Teil oder das gesamte gelöste Salz umfassen.

Die wässrige Lösung hat danach vorzugsweise einen Salzgehalt an von 10 - 30, vorzugsweise 15 - 20 Gew.-%.

Die so behandelte wässrige Lösung kann problemlos weiterverwendet werden. So kann sie einer Elektrolyse zur Gewinnung von Chlor zugeführt oder beispielsweise auch als Kühlmittel eingesetzt werden.

Aus der Lösung kann Salz, insbesondere Kochsalz, hergestellt werden.

Das Verfahren kann problemlos jedem bestehenden Verfahren zur Herstellung von MDA zugefügt werden.

## Patentansprüche

1. Verfahren zur Herstellung von Mischungen von Diphenylmethandiamin und Polyphenylenpolymethylenpolyaminen, umfassend die Schritte,
a) Umsetzung von Anilin mit Formaldehyd in Anwesenheit von Salzsäure,
b) Neutralisation der in Schritt a) entstehenden Reaktionsmischung,
c) Trennung der organischen Phase von der wässrigen Phase,
d) Aufarbeitung der organischen Phase,
e) Aufarbeitung der wässrigen Phase,
**dadurch gekennzeichnet, dass** der Schritt e) mindestens die Schritte
e1) Extraktion der wässrigen Phase mit einem organischen Lösungsmittel,
e2) Strippen der in Schritt e1) erhaltenen wässrigen Phase,
e3) Absorption der aus Schritt e2) erhaltenen Lösung.

2. Verfahren nach Anspruch 1, **dadurch gekennzeichnet, dass** sich an Schritt e3) eine oxidative Behandlung der Lösung anschließt.

3. Verfahren nach Anspruch 1, **dadurch gekennzeichnet, dass** als organisches Lösungsmittel in Schritt e1) Toluol eingesetzt wird.

4. Verfahren nach Anspruch 1, **dadurch gekennzeichnet, dass** das Strippen in Schritt e2) mit Wasserdampf erfolgt.

5. Verfahren nach Anspruch 1, **dadurch gekennzeichnet, dass** als Adsorptionsmittel in Schritt e3) Aktivkohle eingesetzt wird.

6. Verfahren nach Anspruch 1, **dadurch gekennzeichnet, dass** das Adsorptionsmittel in Schritt e3) als Festbett ausgestaltet ist.

7. Verfahren nach Anspruch 2, **dadurch gekennzeichnet, dass** die oxidative Behandlung in Schritt e4) unter Verwendung von Ozon, Peroxiden und/oder Hypochloriden erfolgt.

8. Verfahren nach Anspruch 1, **dadurch gekennzeichnet, dass** die wässrige Phase nach Schritt e3) einen Gehalt an organisch gebundenem Kohlenstoff von kleiner 25 ppm aufweist.

## Claims

1. A process for preparing mixtures of diphenylmethanediamine and polyphenylenepolymethylenepolyamines, which comprises the steps
a) reaction of aniline with formaldehyde in the presence of hydrochloric acid,
b) neutralization of the reaction mixture formed in step a),
c) separation of the organic phase from the aqueous phase,
d) work-up of the organic phase,
e) work-up of the aqueous phase,
wherein step e) comprises at least the steps
e1) extraction of the aqueous phase with an organic solvent,
e2) stripping of the aqueous phase obtained in step e1),
e3) absorption of the solution obtained from step e2).

2. The process according to claim 1, wherein step e3) is followed by an oxidative treatment of the solution.

3. The process according to claim 1, wherein toluene is used as organic solvent in step e1).

4. The process according to claim 1, wherein the stripping in step e2) is carried out using steam.

5. The process according to claim 1, wherein activated carbon is used as adsorbent in step e3).

6. The process according to claim 1, wherein the adsorbent in step e3) is arranged as a fixed bed.

7. The process according to claim 2, wherein the oxidative treatment in step e4) is carried out using ozone, peroxides and/or hypochlorites.

8. The process according to claim 1, wherein the aqueous phase after step e3) has a content of organically bound carbon of less than 25 ppm.

## Revendications

1. Procédé de fabrication de mélanges de diphénylméthane-diamine et de polyphénylènepolyméthylène-polyamines, comprenant les étapes suivantes :
a) la mise en réaction d'aniline avec du formaldéhyde en présence d'acide chlorhydrique,
b) la neutralisation du mélange réactionnel formé à l'étape a),
c) la séparation de la phase organique de la phase aqueuse,
d) le traitement de la phase organique,
e) le traitement de la phase aqueuse,
**caractérisé en ce que** l'étape e) comprend au moins les étapes suivantes :
e1) l'extraction de la phase aqueuse avec un solvant organique,
e2) la réextraction de la phase aqueuse obtenue à l'étape e1),
e3) l'absorption de la solution obtenue à l'étape e2).

2. Procédé selon la revendication 1, **caractérisé en ce que** l'étape e3) est suivie par un traitement oxydatif de la solution.

3. Procédé selon la revendication 1, **caractérisé en ce que** le toluène est utilisé en tant que solvant organique à l'étape e1).

4. Procédé selon la revendication 1, **caractérisé en ce que** la réextraction à l'étape e2) a lieu avec de la vapeur d'eau.

5. Procédé selon la revendication 1, **caractérisé en ce que** du charbon actif est utilisé en tant qu'agent d'adsorption à l'étape e3).

6. Procédé selon la revendication 1, **caractérisé en ce que** l'agent d'adsorption à l'étape e3) est configuré sous la forme d'un lit fixe.

7. Procédé selon la revendication 2, **caractérisé en ce que** le traitement oxydatif à l'étape e4) a lieu en utilisant de l'ozone, des peroxydes et/ou des hypochlorures.

8. Procédé selon la revendication 1, **caractérisé en ce que** la phase aqueuse après l'étape e3) présente une teneur en carbone lié organique inférieure à 25 ppm.
